# EUROPEAN PATENT SPECIFICATION

(11) **EP 4 149 451 B1**
(45) Date of publication and mention of the grant of the patent: **09.12.2026**
(21) Application number: 21805116.7
(22) Date of filing: 12.05.2021
(51) Int. Cl.: A61K 31/145, A61K 31/661, A61K 45/06, A61P 31/12

(54) **CYSTEAMINE, CYSTAMINE OR PHOSPHOCYSTEAMINE FOR USE FOR THE TREATMENT OF SARS-COV-2 INFECTION**
CYSTEAMIN, CYSTAMIN ODER PHOSPHOCYSTAMIN ZUR VERWENDUNG ZUR BEHANDLUNG VON SARS-COV-2-INFEKTIONEN
CYSTÉAMINE, CYSTAMINE OU PHOSPHOCYSTÉAMINE POUR UTILISATION DANS LE TRAITEMENT D'UNE INFECTION PAR LE SARS-COV-2

(30) Priority: 13.05.2020 US 202063024284 P; 14.05.2020 US 202063024974 P
(43) Date of publication of application: 22.03.2023
(73) Proprietor: The Regents of the University of Michigan, Ann Arbor, MI 48109 (US)
(72) Inventor: THOENE, Jess, Ann Arbor, Michigan 48109 (US); GAVIN, Robert, Ann Arbor, Michigan 48109 (US)
(74) Representative: Bals & Vogel Patentanwälte PartGmbB
(86) International application number: PCT/US2021/031987
(87) International publication number: WO 2021/231570

(56) References cited:
- WO-A1-2021/217221
- US-A- 5 554 655
- US-A1- 2007 135 525
- US-A1- 2014 005 088
- US-A1- 2018 003 712
- US-A1- 2020 071 361
- PAUL MCINTOSH: "NovaBiotics announces fast-track repurposing of its experimental drug Nylexa for COVID-19 trials and plans for earliest possible compassionate use - Novabiotics", 13 April 2020 (2020-04-13), XP093157516, Retrieved from the Internet <URL:https://novabiotics.co.uk/novabiotics-announces-fast-track-repurposing-of-its-experimental-drug-nylexa-for-covid-19-trials-and-plans-for-earliest-possible-compassionate-use/>
- NAIONAL INSTITUTES OF HEALTH: "Therapeutic Options Under Investigation | Coronavirus Disease COVID-19", 21 April 2020 (2020-04-21), pages 1, XP093153428, Retrieved from the Internet <URL:https://web.archive.org/web/20200421163941/https://covid19treatmentguidelines.nih.gov/therapeutic-options-under-investigation/>
- ANONYMOUS: "The Best Face and Body Skin-Care Products for 2020 ", SHAPE.COM, 17 April 2020 (2020-04-17), pages 1 - 25, XP055875879, Retrieved from the Internet <URL:https://www.shape.com/lifestyle/beauty-style/best-skin-care-products-2020> [retrieved on 20220103]
- KANDEEL MAHMOUD; AL-NAZAWI MOHAMMED: "Virtual screening and repurposing of FDA approved drugs against COVID-19 main protease", LIFE SCIENCE, PERGAMON PRESS, OXFORD, GB, vol. 251, 3 April 2020 (2020-04-03), GB , XP086144978, ISSN: 0024-3205, DOI: 10.1016/j.lfs.2020.117627

## Description

### CROSS-REFERENCE TO RELATED APPLICATIONS

This application claims priority to the filing dates of United States Provisional Patent Application Serial No. 63/024,284 filed May 13, 2020, and United States Provisional Patent Application Serial No. 63/024,974 filed May 14, 2020.

### FIELD

Provided herein are methods for the treatment and prevention of viral infections (e.g., coronavirus infections (e.g., severe acute respiratory syndrome coronavirus 2 (SARS-CoV-2), etc.), etc.) and diseases (e.g., Coronavirus disease 2019 (COVID-19)) associated therewith by the administration of cysteamine or derivatives thereof to a subject. Any references in the description to methods of treatment refer to the compounds, pharmaceutical compositions and medicaments of the present invention for use in a method for treatment of the human (or animal) body by therapy (or for diagnosis).

### BACKGROUND

Coronavirus disease-2019 (COVID-19), secondary to the severe acute respiratory syndrome coronavirus-2 (SARS-CoV-2), represents an emergent threat to public health, with a quoted mortality rate among inpatients greater than 25%. Containment strategies have been employed to varying degrees and effects in Western nations, now necessitating mitigation strategies in overwhelmed hospitals to effectively address COVID-19. Limited intensive care unit- (ICU) level resources such as mechanical ventilation and other in-hospital resources such as bedding space have played a significant role in the poor clinical outcomes associated with COVID-19.

Paul Mclntosh, "NovaBiotics announces fast-track repurposing of its experimental drug Nylexa for COVID-19 trials and plans for earliest possible compassionate use-NovaBiotics", 2020, URL: https://novabiotics.co.uk/novabiotics-announces-fast-track-repurposing-of-its-experimental-druq-nylexa-for-covid-19-trials-and-plans-for-earliest-possible-compassionate-use/ discloses the repurposing of Nylexa for use in treating secondary bacterial lung infections associated with COVID-19 and suggests availability in patients in the second half of 2020, ahead of any vaccine being available.

US5554655A discloses the effective use of cysteamine, cystamine and phosphocysteamine as agents for the treatment of HIV infections.

National Institute of Health, "Therapeutic Options Under Investigation, Coronavirus Disease COVID-19", 2020, page 1, URL: https://web.archive.orq/web/20200421163941/https://www.covid19treatmentquidelines.nih.qov/t heraputic-options-under-investigation/ discloses potential treatment options for patients infected with COVID-19.

WO2021217221A1 discloses a method for the prophylaxis or treatment of a viral infection in a patient comprising administering a therapeutically effective amount of a combination of a glycoprotein affecting protease and a disulphide bond breaking agent.

The Best Face and Body Skin-Care Products for 2020", 2020, 1-25, Shape.com discloses a face mask comprising an active agent selected from cysteamine and phosphocysteamine.

Kandeel Mahmoud and Al-Nazawi Mohammed, "Virtual screening and repurposing of FDA approved drugs against COVID-19 main protease", Life Science, 2020, 251, DOI: 10.1016/j.lfs.2020.117627 discloses the screening of drugs that may be active against SARS-CoV-2, wherein cysteamine displayed a high docking score.

US2018003712A1 discloses a kit for detecting a virus, a composition for detecting a virus and a method for detecting a virus with high efficiency at low cost within a short period of time.

US2007135525A1 discloses materials and methods for treating various health conditions, including the prevention and/or treatment of a viral infection. Such methods include administering a cysteamine compound to a subject to treat an influenza virus infection.

US2020071361A1 discloses novel peptides that can comprise antimicrobial, antiviral, antifungal or antitumor activity when administered to a subject.

### SUMMARY

The invention is defined in the appended set of claims. Disclosed herein are methods for the treatment and prevention of viral infections (e.g., coronavirus infections (e.g., severe acute respiratory syndrome coronavirus 2 (SARS-CoV-2), etc.), etc.) and diseases (e.g., Coronavirus disease 2019 (COVID-19)) associated therewith by the administration of cysteamine or derivatives thereof to a subject.

In some embodiments, provided herein are methods of treating a subject infected with severe acute respiratory syndrome coronavirus 2 (SARS-CoV-2) comprising administering an effective dose of cysteamine to the subject. In some embodiments, the subject suffers from Coronavirus disease 2019 (COVID-19).

Provided herein is the use of an effective dose of cysteamine for treating a subject infected with severe acute respiratory syndrome coronavirus 2 (SARS-CoV-2).

In some embodiments, provided herein is the use of an effective dose of cysteamine in the manufacture of a medicament for use in a method of treating a subject infected with severe acute respiratory syndrome coronavirus 2 (SARS-CoV-2).

Provided herein are methods of treating a subject infected with severe acute respiratory syndrome coronavirus 2 (SARS-CoV-2) comprising administering cysteamine to the subject.

Provided herein are methods of preventing a subject from becoming infected with severe acute respiratory syndrome coronavirus 2 (SARS-CoV-2), comprising administering cysteamine to the subject (e.g., by way of intranasal, bronchial or systemic administration).

In some embodiments, provided herein is the use of an effective dose of cysteamine for preventing infection of a subject with severe acute respiratory syndrome coronavirus 2 (SARS-CoV-2).

In some embodiments, provided herein is the use of an effective dose of cysteamine in the manufacture of a medicament for use in a method of preventing a subject from being infected with severe acute respiratory syndrome coronavirus 2 (SARS-CoV-2).

In some embodiments, the subject suffers from mild COVID-19. In some embodiments, the subject suffers from moderate COVID-19. In some embodiments, the subject suffers from severe COVID-19. In some embodiments, the effective dose (e.g., oral dose, etc.) comprises 10-500 mg of cysteamine (e.g., 10 mg, 20 mg, 30 mg, 40 mg, 50 mg, 60 mg, 70 mg, 80 mg, 90mg, 100 mg, 150 mg, 200mg, 250 mg, 300 mg, 400 mg, 500 mg, or ranges therebetween (e.g., 25-150 mg of cysteamine, etc.), etc. In some embodiments, cysteamine is administered daily, twice daily, thrice daily, on alternate days, etc. In some embodiments, cysteamine is administered orally, intravenously, or by another suitable route of administration. In some embodiments, the effective dose (e.g., parenteral dose (e.g., IV dose, etc.)) comprises 25-250 mg/kg/day of cysteamine (e.g., 25 mg/kg/day, 50 mg/kg/day, 75 mg/kg/day, 100 mg/kg/day, 150 mg/kg/day, 200 mg/kg/day, 250 mg/kg/day, or ranges therebetween (e.g., 50-150 mg/kg/day, etc.)). In some embodiments, the daily oral or intravenous dose does not exceed 20g/day of cysteamine (e.g., ≤2 g, ≤3 g, ≤4 g, ≤5 g, ≤6 g, ≤7 g, ≤8 g, ≤9 g, ≤10 g ≤20 g). In some embodiments, the subject is first administered an initial dose (e.g., 25 mg/kg/day, 20 mg, etc.) and doses are subsequently increased (e.g., by 10 mg/kg/day, by 25 mg/dose, etc.) to an effective dose.

Disclosed is the co-administration of cysteamine with one or more additional therapeutics. In some embodiments not part of the claimed invention, the cysteamine is co-administered with one or more antiviral agents. In some embodiments not part of the claimed invention, the cysteamine is co-administered with one or more of lopinavir and ritonavir, umifenovir, hydroxychloroquine, remdesivir and favipiravir.

In some embodiments, provided herein are methods comprising: (a) testing a subject or a biological sample from a subject to determine the appropriateness of cysteamine administration to treat/prevent COVID-19; and (b) administering a dose of cysteamine consistent with the methods described herein. In some embodiments, testing comprises a PCR-based test to determine whether the subject is infected with SARS-CoV-2. In some embodiments, methods further comprise: (c) testing a subject or a biological sample from a subject to determine the effectiveness of the cysteamine administration. In some embodiments, methods further comprise: (d) determining a treatment course of action based on the testing of step (a) and/or step (c).

In some embodiments, provided herein are methods of altering the structure of SARS-CoV-2 spike protein comprising contacting the spike protein with cysteamine. In some embodiments, the spike protein is present on the surface of a SARS-CoV-2 viral particle. In some embodiments, the SARS-CoV-2 viral particle is present within a human subject. In some embodiments, contacting the spike protein with cysteamine results in reduction of one or more disulfide bonds within the spike protein.

### BRIEF DESCRIPTION OF THE DRAWINGS

Figure 1. Multiple Sequence Alignment (MSA) of the spike proteins of seven human coronaviruses (229E, NL63, OC43, HKU1, SARS, MERS, and COVID-19).
Figure 2A-B. Bioimaging of Huh-7 cells (A) infected with SARS-CoV-2, and (B) uninfected.

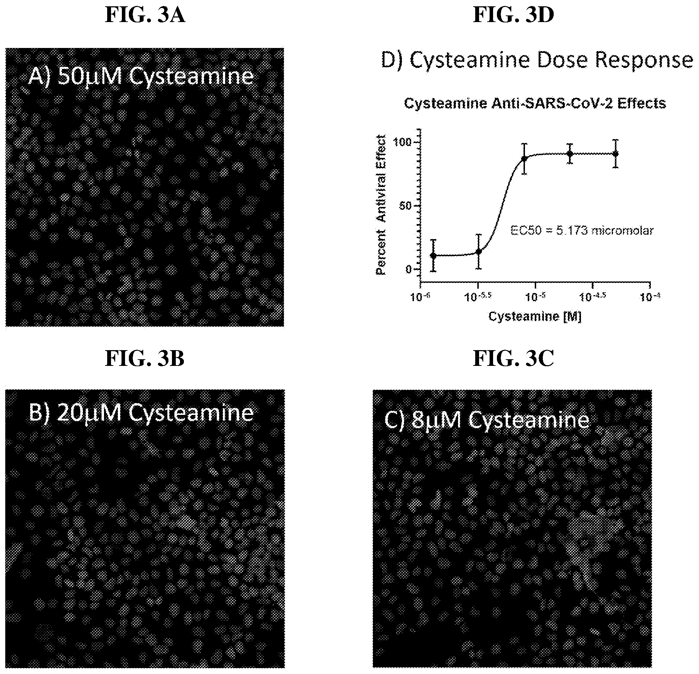

Figure 3A-D. Cysteamine efficacy in the Huh7 cellular infectivity assay with SARS-CoV-2-WA1 strain. Cells are infected at 0.2 MOI and are incubated for 48 hours to allow for the initial infection and propagation to surrounding cells. A) 50 micromolar cysteamine shows 100% elimination of SARS-CoV-2 infection, B) 20 micromolar shows a small percentage of infected cells that have a diminished nucleocapsid staining indicating cysteamine is acting to inhibit viral replication, C) 8 micromolar cysteamine shows reduced viral infection relative to the negative control but syncytia become present and viral staining is increased, D) Concentration response curve for cysteamine with a ~5 micromolar EC50 (concentration at 50% effect). All concentrations of cysteamine (50, 20, 8, 3, 1.5 micromolar) tested were non-cytotoxic indicating an acceptable therapeutic index.

### DEFINITIONS

Although any methods and materials similar or equivalent to those described herein can be used in the practice or testing of embodiments described herein, some preferred methods, compositions, devices, and materials are described herein. However, before the present materials and methods are described, it is to be understood that this invention is not limited to the particular molecules, compositions, methodologies or protocols herein described, as these may vary in accordance with routine experimentation and optimization. It is also to be understood that the terminology used in the description is for the purpose of describing the particular versions or embodiments only, and is not intended to limit the scope of the embodiments described herein.

Unless otherwise defined, all technical and scientific terms used herein have the same meaning as commonly understood by one of ordinary skill in the art to which this invention belongs. However, in case of conflict, the present specification, including definitions, will control. Accordingly, in the context of the embodiments described herein, the following definitions apply.

As used herein and in the appended claims, the singular forms "a", "an" and "the" include plural reference unless the context clearly dictates otherwise. Thus, for example, reference to "a therapeutic" is a reference to one or more therapeutics and equivalents thereof known to those skilled in the art, and so forth.

As used herein, the term "comprise" and linguistic variations thereof denote the presence of recited feature(s), element(s), method step(s), etc. without the exclusion of the presence of additional feature(s), element(s), method step(s), etc. Conversely, the term "consisting of" and linguistic variations thereof, denotes the presence of recited feature(s), element(s), method step(s), etc. and excludes any unrecited feature(s), element(s), method step(s), etc., except for ordinarily-associated impurities. The phrase "consisting essentially of" denotes the recited feature(s), element(s), method step(s), etc. and any additional feature(s), element(s), method step(s), etc. that do not materially affect the basic nature of the composition, system, or method. Many embodiments herein are described using open "comprising" language. Such embodiments encompass multiple closed "consisting of" and/or "consisting essentially of' embodiments, which may alternatively be claimed or described using such language.

As used herein, the term "subject" broadly refers to any animal, including but not limited to, human and non-human animals (e.g., dogs, cats, cows, horses, sheep, poultry, fish, crustaceans, etc.). As used herein, the term "patient" typically refers to a subject that is being treated for a disease or condition.

As used herein, the terms "administration" and "administering" refer to the act of giving a drug, prodrug, or other agent, or therapeutic treatment to a subject or *in vivo, in vitro,* or *ex vivo* cells, tissues, and organs. Exemplary routes of administration to the human body can be by oral administration, parenteral administration (e.g., intravenously, subcutaneously, etc.), etc.

As used herein, the term "effective amount" refers to the amount of a composition sufficient to effect beneficial or desired results. An effective amount can be administered in one or more administrations, applications or dosages and is not intended to be limited to a particular formulation or administration route.

As used herein, the terms "co-administration" and "co-administering" refer to the administration of at least two agent(s) (e.g., cysteamine and one or more additional therapeutics) or therapies to a subject. In some embodiments, the co-administration of two or more agents or therapies is concurrent (e.g., in a single formulation/composition or in separate formulations/compositions). In other embodiments, a first agent/therapy is administered prior to a second agent/therapy. Those of skill in the art understand that the formulations and/or routes of administration of the various agents or therapies used may vary. The appropriate dosage for co-administration can be readily determined by one skilled in the art. In some embodiments, when agents or therapies are co-administered, the respective agents or therapies are administered at lower dosages than appropriate for their administration alone. Thus, co-administration is especially desirable in embodiments where the co-administration of the agents or therapies lowers the requisite dosage of a potentially harmful (e.g., toxic) agent(s), and/or when co-administration of two or more agents results in sensitization of a subject to beneficial effects of one of the agents via co-administration of the other agent.

As used herein, the term "pharmaceutical composition" refers to the combination of an active agent with a carrier, inert or active, making the composition especially suitable for diagnostic or therapeutic use in vitro, in vivo or ex vivo.

The terms "pharmaceutically acceptable" or "pharmacologically acceptable," as used herein, refer to compositions that do not substantially produce adverse reactions, e.g., toxic, allergic, or immunological reactions, when administered to a subject.

As used herein, the term "instructions for administering," and grammatical equivalents thereof, includes instructions for using the compositions contained in a kit for the treatment of conditions (e.g., providing dosing, route of administration, decision trees for treating physicians for correlating patient-specific characteristics with therapeutic courses of action).

As used herein, the term "sample" is used in its broadest sense. In one sense, it is meant to include a specimen or culture obtained from any source, as well as biological and environmental samples. Biological samples may be obtained from animals (including humans) and encompass fluids, solids, tissues, and gases. Suitable samples that may find use in embodiments herein include, but are not limited to: blood, plasma, sera, urine, saliva, cells, cell lysates, tissues, tissue homogenates, purified nucleic acids, stool, vaginal secretions, cerebrospinal fluid, allantoic fluid, water, biofilm, soil, dust, food, beverage, agriculture products, plants, etc.

### DETAILED DESCRIPTION

Disclosed herein are methods for the treatment and prevention of viral infections (e.g., coronavirus infections (e.g., severe acute respiratory syndrome coronavirus 2 (SARS-CoV-2), etc.), etc.) and diseases (e.g., Coronavirus disease 2019 (COVID-19)) associated therewith by the administration of cysteamine or derivatives thereof to a subject.

Cysteamine (2-aminoethanethiol) is an FDA-approved orphan drug for the treatment of cystinosis. It has demonstrated *in vitro* activity against the human immunodeficiency virus (HIV), reducing the disulfide bonds in HIV glycoprotein 120 (GP 120) and thereby inhibiting viral spread (See, U.S. Pat. No. 5,646,189). *In vitro* studies of cysteamine against HIV showed 100% inhibition of cytotoxicity at 100µM concentration (See, U.S. Pat. No. 5,554,655). The assay of cysteamine and cystamine was performed using the CEM human T-lymphocyte cell line as host cells, and the HTLV-III_{B} strain of HIV-1 as the challenge virus. In some embodiments, various cysteamine-related compounds may find use in any embodiments described herein for cysteamine. Suitable cysteamine-related compounds for use in any embodiments herein include cysteamine, cystamine, phosphocysteamine, pharmaceutically acceptable salts thereof, all the hydrated forms of these compounds as well as the anhydrous forms.

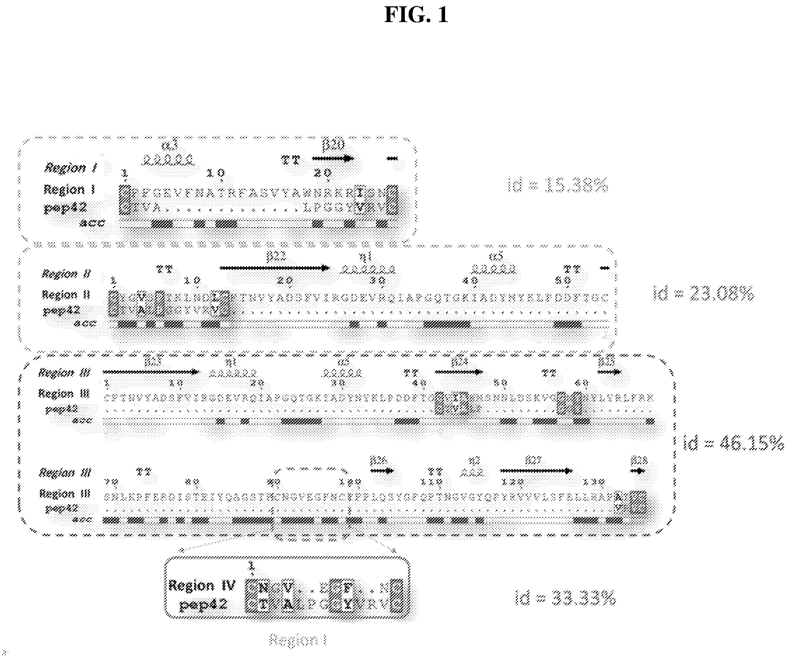

Multiple Sequence Alignment (MSA) was performed between the seven human coronaviruses (229E, NL63, OC43, HKU1, SARS, MERS, and COVID-19) spike proteins (alignment performed by Clustal Omega web server and visualized by ESpript software). Figure 1 highlights results of the analysis (from 'COVID-19 Spike-host cell receptor GRP78 binding site prediction' Ibrahim Ibrahim, Doaa Abdelmalek, Mohamed Elshahat, Abdo Elfiky; DOI: 10.21203/rs.2.24599/v1). The secondary structure for the COVID-19 spike model is displayed at the top of the MSA, and residual surface accessibility is present at the bottom. Alpha helices are shown by helix while arrows show beta-sheets on the top of the MSA. The residues that are surface accessible are in blue, while buried residues are in white at the bottom of the MSA. Identical residues are highlighted in red, while similar residues are highlighted in yellow. The positions of the disulfide bonds are marked by the green numbers below the accessibility rows in the MSA. 13 disulfide bonds are found in the spike protein from which we predict four regions to be the binding site with cell surface GRP78. These four regions of the spike protein, identified with the disulfides numbers 3, 4, 5, and 6 are marked in the MSA with green, blue, magenta, and red dashed lines, respectively.

The effects of cysteamine alone and in association with zidovudine or didanosine on the replication of human immunodeficiency virus type 1 (HIV-1) has been investigated (Beramini et al. J Infect Dis. 1996;174(1):214-218.). More than 90% viral inhibition was obtained by 200 µM cysteamine in lymphocytes and 100 microM cysteamine in macrophages against 4 primary isolates and 2 laboratory strains of HIV-1. Polymerase chain reaction analysis demonstrated that cysteamine interferes with early steps of HIV-1 replication, before proviral DNA formation. The use of cysteamine in conjunction with zidovudine or didanosine brought about an additive antiviral effect without concomitant increases in toxicity. The concentrations of cysteamine that are effective against HIV-1 in vitro have been well tolerated over long periods by patients under treatment for cystinosis, an inherited disorder. These observations indicate that effective doses of cysteamine are tolerable by patients.

Thus, in some embodiments, provided herein are methods for treating/preventing SARS-CoV-2 infection and/or COVID-19 by the administration (e.g., oral, topical, IV, intranasally, intrabronchially, etc.) of an effective amount of cysteamine, cystamine, phosphocysteamine, or a pharmaceutically acceptable salt hereof. Cysteamine is a compound of the formula: 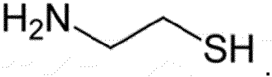 Cystamine is a compound of the formula: 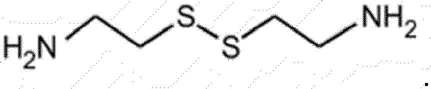 Phosphocysteamine is a compound of the formula:

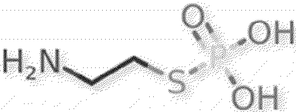

Cysteamine may be prepared from ethanolamine and carbon disulfide via 2-mercaptothiazoline (Gabriel et al, Ber., vol. 31, 2837 (1898); Knorr et al, Ber., vol. 36, 1281 (1903); and Mills, Jr. et al, J. Am. Chem. Soc., vol. 62, 1173 (1940)) or via ethyleneimine (Wenker, J. Am. Chem. Soc., vol. 57, 2328 (1935); Mills, Jr. et al, J. Am. Chem. Soc., vol. 62, 1173 (1940); and Shirley, Preparation of Organic Intermediates, Wiley, NY p. 189 (1951)). Cystamine has the formula

Phosphocysteamine is the phosphorothioester of cysteamine and has the formula:

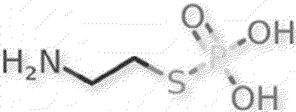

Phosphocysteamine is also known to be useful for the treatment of nephropathic cystinosis (Thoene et al, The Journal of Pediatrics, vol. 96, pp. 1043-1044 (1980); Thoene, in Cooperative Approaches to Research and Development of Orphan Drugs, Alan R. Liss, NY, pp. 157-162 (1985); and Smolin et al, Pediatric Research, vol. 23, pp. 616-620 (1988)).

Cysteamine is known to be safe for use in humans with few side-effects (usually nausea and vomiting) serious enough to require discontinuation of the drug. There is both *in vitro* and *in vivo* data on the safety of cysteamine. Studies on the effect of cysteamine on the growth rate and cloning efficiency of epithelial fibroblasts show no effect on growth rate at concentrations up to 10 µM. These in vitro experiments were performed using fibroblasts which are not protected as are cornified epithelial cells, in vivo, as found in the oral, vaginal and anal cavities. Studies have also been performed on topical cysteamine for use in the treatment of the keratitis of cystinosis. In this formulation, aqueous cysteamine at concentrations of 0.1% and 0.5%, which correspond to 10 mM and 50 mM, respectively, were used. These concentrations are effective in reducing the cystine crystals found in the corneas of patients with nephropathic cystinosis, and the drug is well-tolerated in chronic use via this modality. Rabbit studies have shown both concentrations to be safe as evaluated via the Draize test (Kaiser-Kupfer, M. et al, Archives of Ophthalmology, vol. 108, pp. 689-693 (1990); and Kaiser-Kupfer, M. et al, New Eng. J. Med., vol. 316, pp. 775-779 (1987)).

Provided herein are compositions (e.g., comprising cysteamine) and methods (e.g., administering cysteamine or co-administering cysteamine with additional therapies) for the treatment of SARS-CoV-2 infection, COVID-19, and symptoms/conditions arising therefrom (e.g., lung damage, organ failure, etc.). In some embodiments, provided herein is the use of unit doses of cysteamine in the range of 10-250 mg, repeated at intervals (e.g., 6 hours, 12 hours, 24 hours, 48 hours, etc.) as required. In some embodiments, dose may be administered up to twenty doses (e.g., 2, 3, 4, 5, 6, 7, 8, 9, 10, 11, 12, 13, 14, 15, 16, 17, 18, 19, 20 or ranges therebetween). In some embodiments, dose administration may be continued beyond 20 doses. In some embodiments, a maximum total dose/day is, for example, 2-10g (e.g., 2, g, 3, g, 4 g, 5 g, 6 g, 7 g, 8 g, 9 g 10 g, or more or ranges therebetween). In some embodiments, disclosed herein is the combination of cysteamine with antiviral drugs, other immunosuppressive therapies, etc. In some embodiments, cysteamine is administered orally, parenterally (e.g., intravenously, subcutaneously, intramuscularly, etc.), or by any suitable route of administration. In some embodiments, dosing is guided by laboratory and/or clinical studies.

The compositions and methods herein find use in the treatment of a viral infection and/or diseases, conditions, or symptoms arising therefrom. In some instances, the viral target which mediates the viral infection and/or disease, condition or symptom arising therefrom is one that shows significant reliance on disulfide bonds to promote infection and spread. Cysteamine is administered (alone or co-administered with other agents, including but not limited to urea, guanidine or other chaotropic agents, e.g., in a nasal spray) for the treatment of a viral infection and/or diseases, conditions, or symptoms arising therefrom, e.g., SARS-CoV-2 mediated infections, diseases, conditions and/or symptoms.

In some embodiments, a subject that is infected with SARS-CoV-2 (e.g., tested positive for SARS-CoV-2) is treated with cysteamine to prevent/treat symptoms and complications resulting therefrom, In some embodiments, a subject suffers from COVID-19 as a result of a SARS-CoV-2 infection.

COVID-19 may be classified as "mild," "moderate," "severe," and "critical." A mild case of COVID-19 (which may develop into a more severe case, but might not) typically consists of a low/moderate fever, respiratory symptoms, cough, aches, and pains. An individual patient might exhibit only a subset of these symptoms. Moderate COVID-19 is defined as a subject displaying evidence of pulmonary infiltrates (by chest X-ray or CT scan), but without a need for supplemental oxygen (or more than the patient's baseline oxygen requirement if using home oxygen). Symptoms of being moderately ill with COVID-19 may include coughing, fever above 37.8°C, chills, shortness of breath, dehydration, and increased tiredness. A severe case of COVID-19 will include the preceding symptoms as well as inflammation in the lungs (e.g., leading to cytokine release), pneumonia, low oxygen levels in the blood, trouble breathing, pain or pressure in the chest, confusion, inability to arouse, difficulty eating or drinking, and bluish lips/face. A critical case of COVID-19 may include severe pneumonia, ARDS (acute respiratory distress syndrome), sepsis, and organ failure.

In some embodiments, cysteamine is administered to a subject suffering from COVID-19. In some embodiments, cysteamine is administered to a subject suffering from moderate COVID-19. In some embodiments, cysteamine is administered to a subject suffering from severe COVID-19.

In some embodiments, a subject is critically ill from COVID-19. In some embodiments, a critically ill patient exhibits one or more of ICU utilization, pneumonitis (e.g., physiologic signs of pneumonitis), bilateral infiltrates (e.g., radiographic evidence of infiltrates), respiratory failure, septic shock, increased respiratory rate (e.g., >20, >25, >30, >35, >40 per minute), low oxygen saturation (e.g., <95%, <94%, <93%, <92%, <91%, or <90% in ambient air), respiratory failure requiring mechanical ventilation, shock of any form (e.g., requiring the use of a vasopressor medication), or organ failure (e.g., multiorgan failure).

In some embodiments, a subject suffering from COVID-19 exhibits mild pneumonitis (e.g., no pulmonary infiltrates, mild symptoms, etc.).

In some embodiments, a subject has a positive test for active SARS-CoV-2 infection. In some embodiments, a subject exhibits signs/symptoms of COVID-19, such as fever (e.g., >100.4), cough, aches/pains, chills, shortness of breath, dehydration, increased tiredness.

In some embodiments, a subject is not critically ill, but at moderate to high risk of the disease advancing to ICU utilization and/or COVID-19 mortality. In some embodiments, a subject is not critically ill, but exhibits symptoms that warrant hospital admission.

In some embodiments, a subject is not critically ill, but suffering from COVID-19, exhibits pneumonitis, and is at low risk of the disease advancing to ICU utilization and/or COVID-19 mortality.

In some embodiments, provided herein are methods of preventing infection of a subject with severe acute respiratory syndrome coronavirus 2 (SARS-CoV-2) comprising administering an effective dose of cysteamine to the subject. In some embodiments, cysteamine is administered to the nasal cavity of the subject. In some embodiments, cysteamine is formulated for topical administration. In some embodiments, the topical formulation comprises a nasal cream, nasal drops, or a nasal spray. In some embodiments, administering cysteamine reduces the effectiveness of SARS-CoV-2 entering the nasal epithelium, replicating in the nasal epithelium, and/or migrating from the nasal epithelium. In some embodiments, provided herein is the use of an effective dose of cysteamine for preventing infection of a subject with severe acute respiratory syndrome coronavirus 2 (SARS-CoV-2). In some embodiments, provided herein is the use of an effective dose of cysteamine in the manufacture of a medicament for use in a method of preventing a subject from being infected with severe acute respiratory syndrome coronavirus 2 (SARS-CoV-2).

Cysteamine bitartrate immediate release is US FDA approved as Cystagon (Mylan Pharma) in 50mg and 150 mg capsules. Procysbi, the delayed release form, (Horizon) is supplied in 25mg and 75 mg microbeads, which decreases the amount of API per unit weight, thereby increasing by 50% the number of capsules required for a given dose.

Cysteamine has been administered intravenously (Thoene,et al JCI, 1976).

Cysteamine can be administered intrabronchially, e.g. by way of bronchial inhaler, or other means. In some embodiments, cysteamine is formulated as a liquid or micronized powder, which permit achieving very high intra-bronchial concentrations.

Cysteamine is marketed as a topical 5% cream to treat pigmentary skin lesions. In some embodiments, topical cysteamine is administered as a nasal cream, nasal drops or nasal spray for example, to reduce the effectiveness of SARS-CoV-2 in entering the nasal epthelium or replicating therein or migrating therefrom, which a recent report identifies as a prime point of entry (Sungnak, W., Huang, N., Bécavin, C. et al. SARS-CoV-2 entry factors are highly expressed in nasal epithelial cells together with innate immune genes. Nat Med (2020). https://doi.org/10.1038/s41591-020-0868-6).

In some embodiments, cysteamine is provided in 10-500 mg doses (e.g., 10 mg, 20 mg, 30 mg, 40 mg, 50 mg, 60 mg, 70 mg, 80 mg, 90 mg, 100 mg, 110 mg, 120 mg, 130 mg, 140 mg, 150 mg, 160 mg, 170 mg, 180 mg, 190 mg, 200 mg, 210 mg, 220 mg, 230 mg, 240 mg, 250 mg, 260 mg, 270 mg, 280 mg, 290 mg, 300 mg, 310 mg, 320 mg, 330 mg, 340 mg, 350 mg, 360 mg, 370 mg, 380 mg, 390 mg, 400 mg, 410 mg, 420 mg, 430 mg, 440 mg, 450 mg, 460 mg, 470 mg, 480 mg, 490 mg, 500 mg, and ranges therebetween (e.g., 50-150 mg)). In some embodiments, the dose is adjusted based on the effectiveness and/or side effects of initial dosing. For example, if the initial dose is/appears effective and/or side effects are/appear significant, the dose may be reduced (e.g., 10-100 mg (e.g., 10 mg, 20 mg, 30 mg, 40 mg, 50 mg, 60 mg, 70 mg, 80 mg, 90 mg, 100 mg, and ranges therebetween (e.g., 30-50 mg)). If the initial dose is/appears ineffective and/or side effects are/appear insignificant, the dose may be increased. In some embodiments, an initial dose of cysteamine is provided and ramped up to a final dose with increments of, for example, 10mg/kg/d at intervals (e.g., 2 week intervals). In some embodiments, cysteamine is administered (e.g., daily, twice daily, thrice daily, on alternate days, weekly, etc.) for a set time span (e.g., 1 week, 2 weeks, 3 weeks, 4 weeks, or more), of until symptoms or testing demonstrate the effectiveness of the treatment.

In some embodiments, dosing is guided by clinical and/or laboratory studies.

In some embodiments, cysteamine is administered as a nanowafer. Nanowafers are constructs of a suitable biocompatible polymeric material, e.g., poly(vinyl alcohol) (MW 146,000, 87- 89% hydrolyzed), containing cysteamine and fabricated in poly(dimethylsiloxane) (PDMS) imprints containing vertical of 500 nm × 500 nm x 500 nm. Further details may be found in Marcano, D et.al. "Synergistic Cysteamine Delivery Nanowafer as an Efficacious Treatment Modality for Corneal Cystinosis," Mol. Pharmaceutics (2016) 13: 3468-3477

Methods are disclosed for the treatment or prevention of viral infection by the co-administration of cysteamine with one or more additional therapeutics or therapies (e.g., for the treatment of viral infection (e.g., SARS-CoV-2), for treatment of COVID-19, etc.).

It is disclosed that cysteamine may be co-administered with a suitable antiviral agent. Exemplary, although non-limiting, antiviral agents may be selected from abacavir, acyclovir, adefovir, amantadine, ampligen, amprenavir, arbidol, atazanavir, atripla, balavir, baloxavir marboxil, biktarvy, boceprevir, cidofovir, cobicistat, combivir, daclatasvir, darunavir, delavirdine, descovy, didanosine, docosanol, dolutegravir, doravirine, ecoliever, edoxudine, efavirenz, elvitegravir, emtricitabine, enfuvirtide, entecavir, etravirine, famciclovir, favipravir fomivirsen, fosamprenavir, foscarnet, fosfonet, a fusion inhibitor, ganciclovir, ibacitabine, ibalizumab, idoxuridine, imiquimod, imunovir, indinavir, inosine, an integrase inhibitor, interferon type i, interferon type ii, interferon type iii, interferon, lamivudine, letermovir, lopinavir, loviride, maraviroc, methisazone, moroxydine, nelfinavir, nevirapine, nexavir, nitazoxanide, norvir, nucleoside analogues, oseltamivir, peginterferon alfa-2a, peginterferon alfa-2b, penciclovir, peramivir, pleconaril, podophyllotoxin, a protease inhibitor, pyramidine, raltegravir, remdesivir, a reverse transcriptase inhibitor, ribavirin, rilpivirine, rimantadine, ritonavir, saquinavir, simeprevir, sofosbuvir, stavudine, a synergistic enhancer, telaprevir, telbivudine, tenofovir alafenamide, tenofovir disoproxil, tenofovir, tipranavir, trifluridine, trizivir, tromantadine, truvada, valaciclovir, valganciclovir, vicriviroc, vidarabine, viramidine, zalcitabine, zanamivir (relenza), zidovudine, etc.

It is disclosed that cysteamine may be co-administered with a combination of lopinavir and ritonavir. In some embodiments, cysteamine is co-administered with alpha-interferon. In some embodiments, cysteamine is co-administered with an oral combination of lopinavir and ritonavir and nebulized alpha-interferon.

It is disclosed that cysteamine may be co-administered with umifenovir.

It is disclosed that cysteamine may be co-administered with hydroxychloroquine.

It is disclosed that cysteamine may be co-administered with remdesivir.

It is disclosed that cysteamine may be co-administered with glucocorticoids (e.g., high doses of glucocorticoids).

It is disclosed that cysteamine may be co-administered with one or more suitable immunosuppressant drugs. Exemplary, although non-limiting, immunosuppressant drugs may be selected from corticosteroids (e.g., prednisone, budesonide, prednisolone, etc.), janus kinase inhibitors (e.g., tofacitinib, etc.), calcineurin inhibitors (e.g., cyclosporine, tacrolimus, mTOR inhibitors (e.g., sirolimus, everolimus, etc.), IMDH inhibitors (e.g., azathioprine, leflunomide, mycophenolate, etc.), biologics (e.g., abatacept, adalimumab, anakinra, certolizumab, etanercept, golimumab, infliximab, ixekizumab, natalizumab, rituximab, secukinumab, tocilizumab, ustekinumab, vedolizumab, etc.), monoclonal antibodies (e.g., basiliximab, daclizumab), etc.), etc.

Cysteamine is incorporated into a face mask. In such instances, cysteamine or compositions comprising the same (e.g., a cysteamine solution) may be employed with any type of face mask of interest. Face masks with which compositions of the invention may be employed include both sterile and non-sterile facemasks. In some instances, the facemasks are formed of a panel, such as flat, rectangular panel, of a filtering material, e.g., fabric, which covers at least a portion of the face of the wearer, e.g., the mouth and/or nose of the wearer, with loops, ties or strings attached to the corners of the rectangular panel, e.g., to secure the mask to the face by interaction with the ears, tying behind the head, etc. In some instances, the face masks are surgical face masks. In some instances, association of cysteamine with the mask greatly improves the mask effectiveness in preventing SarsCoV2 infection. A cysteamine composition, e.g., solution, such as a nasal solution, could be sprayed onto a mask, e.g., at or before the time of use, or incorporated into a mask, e.g., such as made with cysteamine in the mask fabric, sealed in a moisture proof bag until use, and then discarded. Such embodiments when employed with nasal compositions may, in some instances, reduce the number of cysteamine nasal administrations, e.g., nose sprays, required per day, and they could be used as PPE in areas where the endemic rate is high, and be employed by health care workers until vaccination is available.

In some embodiments, methods are provided for testing, assessing, quantifying, qualifying, evaluating, etc. one or more signs, symptoms, biomarkers, etc. of SARS-CoV-2 infection, COVID-19, etc. Any techniques described herein (e.g., chest x-ray, qPCR, ELISA, etc.) for the detection of biomarkers, symptoms, etc. are within the scope herein.

In some embodiments, methods herein comprise testing a subject for the presence of one or more symptoms and/or biomarkers of SARS-CoV-2 infection, COVID-19, etc. and then administering a dose of cysteamine (or co-administering) based on the results of the testing.

In some embodiments, methods herein comprise administering a dose of cysteamine and subsequently testing a subject for the presence of one or more symptoms and/or biomarkers of SARS-CoV-2 infection, COVID-19, CRS, etc. and to determine the success of the treatment.

In some embodiments, provided herein are multiple rounds of testing and treating (e.g., test-treat-test, treat-test-treat, etc.).

Some embodiments herein comprise a step of identifying a subject as being infected with SARS-CoV-2 using a suitable assay or kit. In particular embodiments, a PCR-based assay is used to test a biological sample (e.g., blood, saliva, etc.) from a subject for SARS-CoV-2 infection.

Some embodiments herein comprise performing one or more tests to assess whether a subject suffers from COVID-19. In some embodiments, one or more tests is performed to determine if a subject is critically ill with COVID-19, not critically ill but at high risk for disease advancement, not critically ill and at low risk for disease advancement, suffering from mild COVID-19, suffering from moderate COVID-19, suffering from severe COVID-19, etc. In some embodiments, a treatment course of action (e.g., comprising administering cysteamine) is selected based on testing using the assays, biomarkers, symptoms, etc. described herein. In some embodiments, the efficacy/success of a treatment course of action (e.g., comprising administering cysteamine) is measured/assessed based on testing using the assays, biomarkers, symptoms, etc. described herein. Any assays, biomarkers, or symptoms described herein may be used in embodiments herein to determine a treatment course of action (e.g., whether to administer cysteamine, dose/regimen of cysteamine, what to co-administer with cysteamine, etc.) and/or assess the success of a treatment (e.g., whether to continue/discontinue, whether to alter dose, whether to begin/continue/discontinue a co-therapy, etc.).

### EXPERIMENTAL

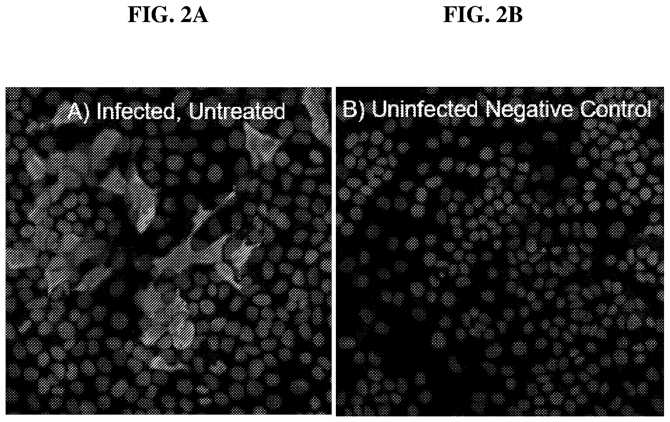

Experiments were conducted during development of embodiments herein using a fully developed high-content screening pipeline to screen approved drugs against SARS Cov-2, the causative agent of COVID -19. A high throughput screen of FDA-approved drugs was conducted to determine the efficacy in a SARS-CoV-2 infectivity assay. This high content bioimaging-based assay has proven to be highly robust and informative, and clearly differentiates between infected and uninfected cells (Fig. 2). This high-content screen identifies individual agents effective in reducing viral infectivity or replication by imaging individual SARS Cov-2 infected cells. A screen of 1,200 compounds was conducted through the validated assay platform. Remdesivir, a viral replication inhibitor, was selected as a positive control, which results in 100% efficacy.

Experiments conducted during development of embodiments herein using the validated assay demonstrate that cysteamine has nearly 100% efficacy in preventing SARS-CoV-2 infection in Huh-7 cells (Fig. 3). Cysteamine was evaluated for its antiviral effects at 5 doses with 9 replicates at each dose for a total of 45 independent experiments. Automated image analysis was performed to quantitate the number and percentage of infected cells and that was normalized to the infected and vehicle (DMSO) treated wells and the uninfected and vehicle treated wells representing 0-100% efficacy, as shown on the y-axis dose response plot for cysteamine anti-SARS-CoV-2 (Fig. 3).

### REFERENCES

(1) Prescott, L. F., R. W. Newton, C. P. Swainson, N. Wright, A. R. W. Forrest, and H. Matthew. 1974. Successful treatment of severe paracetamol overdosage with cysteamine. Lancet. 1: 588-592.)
(2) Thoene JG, Oshima RG, Crawhall JC, Olson DL, Schneider JA. Cystinosis. Intracellular cystine depletion by aminothiols in vitro and in vivo. J Clin Invest. 1976 Jul;58(1):180-9. PubMed PMID: 932205; PubMed Central PMCID:PMC333169.)
(3) Gahl WA, Reed GF, Thoene JG, et al. Cysteamine therapy for children with nephropathic cystinosis. N Engl J Med. 1987;316(16):971-977. doi:10.1056/NEJM198704163161602
(4) Corden BJ, Schulman JD. Schneider JA, Thoene JG.. Adverse reactions to oral cysteamine use in nephropathic cystinosis . Dev Pharmacol Ther 1981; 3:25-30.
(5) Wenner WJ, Murphy JL: The effects of cysteamine on the upper gastrointestinal tract of children with cystinosis. Pediatr Nephrol 11:600, 1997. [9323287
6) Krischock L, Horsfield C, D'Cruz D, Rigden SP. Drug-induced lupus and antiphospholipid syndrome associated with cysteamine therapy. Nephrol Dial Transplant. 2009 Jun;24(6):1997-9. doi: 10.1093/ndt/gfp128. Epub 2009 Mar 26. PubMed PMID: 19324914.
7) Ahmad ZP, Johnstone LM, Walker AM. Cystinosis and lupus erythematosus: coincidence or causation. Pediatr Nephrol. 2010 Aug;25(8):1543-6. doi: 10.1007/s00467-010-1470-9. Epub 2010 Feb 27. Review. PubMed PMID: 20191368.
8) 416. Besouw MT, Bowker R, Dutertre JP, Emma F, Gahl WA, Greco M, Lilien MR, McKiernan J, Nobili F, Schneider JA, Skovby F, van den Heuvel LP, Van't Hoff WG, Levtchenko EN. Cysteamine toxicity in patients with cystinosis. J Pediatr. 2011 Dec;159(6):1004-11. doi: 10.1016/j.jpeds.2011.05.057. Epub 2011 Jul 23. PubMed PMID: 21784456.

In at least some of the previously described embodiments, one or more elements used in an embodiment can interchangeably be used in another embodiment unless such a replacement is not technically feasible. It will be appreciated by those skilled in the art that various other omissions, additions and modifications may be made to the methods and structures described above without departing from the scope of the claimed subject matter. All such modifications and changes are intended to fall within the scope of the subject matter, as defined by the appended claims.

It will be understood by those within the art that, in general, terms used herein, and especially in the appended claims (*e.g*., bodies of the appended claims) are generally intended as "open" terms (*e.g*., the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to embodiments containing only one such recitation, even when the same claim includes the introductory phrases "one or more" or "at least one" and indefinite articles such as "a" or "an" (*e.g*., "a" and/or "an" should be interpreted to mean "at least one" or "one or more"); the same holds true for the use of definite articles used to introduce claim recitations. In addition, even if a specific number of an introduced claim recitation is explicitly recited, those skilled in the art will recognize that such recitation should be interpreted to mean at least the recited number (*e.g*., the bare recitation of "two recitations," without other modifiers, means at least two recitations, or two or more recitations). Furthermore, in those instances where a convention analogous to "at least one of A, B, and C, etc." is used, in general such a construction is intended in the sense one having skill in the art would understand the convention (*e.g.,* " a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (*e.g*., " a system having at least one of A, B, or C" would include but not be limited to systems that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A" or "B" or "A and B."

In addition, where features or aspects of the disclosure are described in terms of Markush groups, those skilled in the art will recognize that the disclosure is also thereby described in terms of any individual member or subgroup of members of the Markush group.

As will be understood by one skilled in the art, for any and all purposes, such as in terms of providing a written description, all ranges disclosed herein also encompass any and all possible sub-ranges and combinations of sub-ranges thereof. Any listed range can be easily recognized as sufficiently describing and enabling the same range being broken down into at least equal halves, thirds, quarters, fifths, tenths, etc. As a non-limiting example, each range discussed herein can be readily broken down into a lower third, middle third and upper third, etc. As will also be understood by one skilled in the art all language such as "up to," "at least," "greater than," "less than," and the like include the number recited and refer to ranges which can be subsequently broken down into sub-ranges as discussed above. Finally, as will be understood by one skilled in the art, a range includes each individual member. Thus, for example, a group having 1-3 articles refers to groups having 1, 2, or 3 articles. Similarly, a group having 1-5 articles refers to groups having 1, 2, 3, 4, or 5 articles, and so forth.

Although the foregoing invention has been described in some detail by way of illustration and example for purposes of clarity of understanding, it is readily apparent to those of ordinary skill in the art in light of the teachings of this invention that certain changes and modifications may be made thereto without departing from the scope of the appended claims.

Accordingly, the preceding merely illustrates the principles of the invention. It will be appreciated that those skilled in the art will be able to devise various arrangements which, although not explicitly described or shown herein, embody the principles of the invention and are included within its scope. Furthermore, all examples and conditional language recited herein are principally intended to aid the reader in understanding the principles of the invention and the concepts contributed by the inventors to furthering the art, and are to be construed as being without limitation to such specifically recited examples and conditions. Moreover, all statements herein reciting principles, aspects, and embodiments of the invention as well as specific examples thereof, are intended to encompass both structural and functional equivalents thereof. Additionally, it is intended that such equivalents include both currently known equivalents and equivalents developed in the future, i.e., any elements developed that perform the same function, regardless of structure. Moreover, nothing disclosed herein is intended to be dedicated to the public regardless of whether such disclosure is explicitly recited in the claims.

The scope of the present invention, therefore, is not intended to be limited to the exemplary embodiments shown and described herein. Rather, the scope of present invention is embodied by the appended claims.

## Claims

1. A composition comprising an effective dose of a solo active agent selected from cysteamine, cystamine and phosphocysteamine as an antiviral for use in the treatment of severe acute respiratory syndrome coronavirus 2 (SARS-CoV-2) in a subject.

2. The composition for use according to claim 1, wherein the subject suffers from Coronavirus disease 2019 (COVID-19), particularly wherein the subject suffers from mild COVID-19 or particularly wherein the subject suffers from moderate COVID-19 or particularly wherein the subject suffers from severe COVID-19; wherein mild COVID-19 is **characterized by** one or more of a low/moderate fever, respiratory symptoms, cough, aches, and pains; wherein moderate COVID-19 is **characterized by** evidence of pulmonary infiltrates without a need for supplemental oxygen, coughing, fever above 37.8°C, chills, shortness of breath, dehydration, and increased tiredness; wherein severe COVID-19 is **characterized by** one or more of inflammation in the lungs, pneumonia, low oxygen levels in the blood, trouble breathing, pain or pressure in the chest, confusion, inability to arouse, difficulty eating or drinking, and bluish lips/face.

3. The composition for use according to one of claims 1-2, wherein the effective dose comprises 10-500 mg of active agent, particularly wherein the effective dose comprises 25-150 mg of active agent.

4. The composition for use according to one of claims 1-3, wherein the active agent is administered daily, particularly wherein the active agent is administered twice daily.

5. The composition for use according to one of claims 1-4, wherein the active agent is administered orally, and/or wherein the active agent is administered intravenously.

6. The composition for use according to one of claims 1-5, wherein the effective dose comprises 25-250 mg/kg/day of the active agent, and/or wherein the daily does not exceed 20g/day of the active agent, and/or wherein the subject is first administered an initial dose and doses are subsequently increased to an effective dose.

7. The composition for use according to one of the claims 1-6, wherein the subject has been tested to determine the appropriateness of cysteamine administration to treat/prevent COVID-19, wherein if appropriate the subject is administered with a dose of an active agent.

8. The composition for use according to claim 7, wherein testing comprises a PCR-based test to determine whether the subject is infected with SARS-CoV-2.

9. The composition for use according to claim 7, wherein the subject is tested to determine the effectiveness of the active agent administration.

10. A substance comprising a solo active agent selected from cysteamine, cystamine and phosphocysteamine as an antiviral for use in a method of treating a subject infected with severe acute respiratory syndrome coronavirus 2 (SARS-CoV-2) wherein severe COVID-19 is **characterized by** one or more of inflammation in the lungs, pneumonia, low oxygen levels in the blood, trouble breathing, pain or pressure in the chest, confusion, inability to arouse, difficulty eating or drinking, and bluish lips/face.

11. The substance for use according to claim 10, wherein contacting SARS-CoV-2 spike protein with the active agent alters the structure of SARS-CoV-2 spike protein, particularly wherein contacting the spike protein with the active agent results in reduction of one or more disulfide bonds within the spike protein.

12. The substance for use according to claim 11, wherein the spike protein is present on the surface of a SARS-CoV-2 viral particle, particularly wherein the SARS-CoV-2 viral particle is present within a human subject.

13. A composition for use as a medicament comprising an effective dose of a solo active agent selected from cysteamine, cystamine and phosphocysteamine as an antiviral for use in the prevention of infection of a subject with severe acute respiratory syndrome coronavirus 2 (SARS-CoV-2), particularly wherein the active agent is administered to the nasal cavity of the subject, particularly wherein the active agent is formulated for topical administration, particularly wherein the topical formulation comprises a nasal cream, nasal drops, or a nasal spray.

14. The composition for use of claim 13 wherein said administering reduces the effectiveness of SARS-CoV-2 entering the nasal epithelium, replicating in the nasal epithelium, and/or migrating from the nasal epithelium.

15. A face mask comprising a panel of a filtering material and an active agent selected from cysteamine, cystamine and phosphocysteamine incorporated into the panel.

## Patentansprüche

1. Eine Zusammensetzung, umfassend eine wirksame Dosis eines einzelnen Wirkstoffs, ausgewählt aus Cysteamin, Cystamin und Phosphocysteamin, als antivirales Mittel zur Verwendung bei der Behandlung des schweren akuten respiratorischen Syndrom-Coronavirus 2 (SARS-CoV-2) bei einem Subjekt.

2. Zusammensetzung zur Verwendung gemäß Anspruch 1, wobei das Subjekt an der Coronavirus-Krankheit 2019 (COVID-19) leidet, insbesondere wobei das Subjekt an einer leichten COVID-19-Erkrankung leidet oder insbesondere wobei das Subjekt an einer mittelschweren COVID-19-Erkrankung leidet oder insbesondere wobei das Subjekt an einer schweren COVID-19-Erkrankung leidet; wobei eine leichte COVID-19-Erkrankung durch eines oder mehrere der folgenden Merkmale gekennzeichnet ist: leichtes bis mäßiges Fieber, Atemwegsbeschwerden, Husten, Gliederschmerzen und Schmerzen; wobei eine mittelschwere COVID-19-Erkrankung durch Anzeichen von Lungeninfiltraten ohne Bedarf an zusätzlichem Sauerstoff, Husten, Fieber über 37,8 °C, Schüttelfrost, Atemnot, Dehydrierung und erhöhte Müdigkeit gekennzeichnet ist; wobei eine schwere COVID-19-Erkrankung durch eines oder mehrere der folgenden Merkmale gekennzeichnet ist: Entzündung der Lunge, Lungenentzündung, niedrige Sauerstoffwerte im Blut, Atembeschwerden, Schmerzen oder Druckgefühl in der Brust, Verwirrtheit, Unfähigkeit, aus dem Schlaf geweckt zu werden, Schwierigkeiten beim Essen oder Trinken sowie bläuliche Lippen/Gesicht.

3. Zusammensetzung zur Verwendung gemäß einem der Ansprüche 1 bis 2, wobei die wirksame Dosis 10-500 mg Wirkstoff umfasst, insbesondere wobei die wirksame Dosis 25-150 mg Wirkstoff umfasst.

4. Zusammensetzung zur Verwendung gemäß einem der Ansprüche 1 bis 3, wobei der Wirkstoff täglich verabreicht wird, insbesondere wobei der Wirkstoff zweimal täglich verabreicht wird.

5. Zusammensetzung zur Verwendung gemäß einem der Ansprüche 1 bis 4, wobei der Wirkstoff oral verabreicht wird und/oder wobei der Wirkstoff intravenös verabreicht wird.

6. Zusammensetzung zur Verwendung gemäß einem der Ansprüche 1 bis 5, wobei die wirksame Dosis 25-250 mg/kg/Tag des Wirkstoffs umfasst und/oder wobei die Tagesdosis 20 g/Tag des Wirkstoffs nicht überschreitet und/oder wobei dem Subjekt zunächst eine Anfangsdosis verabreicht wird und die Dosen anschließend auf eine wirksame Dosis erhöht werden.

7. Zusammensetzung zur Verwendung gemäß einem der Ansprüche 1 bis 6, wobei das Subjekt getestet wurde, um die Eignung einer Cysteamin-Verabreichung zur Behandlung/Vorbeugung von COVID-19 zu bestimmen, wobei dem Subjekt, falls geeignet, eine Dosis eines Wirkstoffs verabreicht wird.

8. Zusammensetzung zur Verwendung gemäß Anspruch 7, wobei die Untersuchung einen PCR-basierten Test umfasst, um festzustellen, ob das Subjekt mit SARS-CoV-2 infiziert ist.

9. Zusammensetzung zur Verwendung gemäß Anspruch 7, wobei das Subjekt getestet wird, um die Wirksamkeit der Verabreichung des Wirkstoffs zu bestimmen.

10. Eine Substanz, umfassend einen einzelnen Wirkstoff, ausgewählt aus Cysteamin, Cystamin und Phosphocysteamin, als antivirales Mittel zur Verwendung in einem Verfahren zur Behandlung eines Subjekts, das mit dem schweren akuten respiratorischen Syndrom-Coronavirus 2 (SARS-CoV-2) infiziert ist, wobei eine schwere COVID-19-Erkrankung durch eines oder mehrere der folgenden Merkmale gekennzeichnet ist: Entzündung der Lunge, Lungenentzündung, niedrige Sauerstoffwerte im Blut, Atembeschwerden, Schmerzen oder Druckgefühl in der Brust, Verwirrtheit, Unfähigkeit, aus dem Schlaf geweckt zu werden, Schwierigkeiten beim Essen oder Trinken sowie bläuliche Lippen/Gesicht.

11. Substanz zur Verwendung gemäß Anspruch 10, wobei das Inkontaktbringen des SARS-CoV-2-Spike-Proteins mit dem Wirkstoff die Struktur des SARS-CoV-2-Spike-Proteins verändert, insbesondere wobei das Inkontaktbringen des Spike-Proteins mit dem Wirkstoff zu einer Reduktion einer oder mehrerer Disulfidbrücken innerhalb des Spike-Proteins führt.

12. Substanz zur Verwendung gemäß Anspruch 11, wobei das Spike-Protein auf der Oberfläche eines SARS-CoV-2-Viruspartikels vorhanden ist, insbesondere wobei das SARS-CoV-2-Viruspartikel in einem menschlichen Subjekt vorhanden ist.

13. Eine Zusammensetzung zur Verwendung als Arzneimittel, umfassend eine wirksame Dosis eines einzelnen Wirkstoffs, ausgewählt aus Cysteamin, Cystamin und Phosphocysteamin, als antivirales Mittel zur Verwendung bei der Vorbeugung einer Infektion eines Subjekts mit dem schweren akuten respiratorischen Syndrom-Coronavirus 2 (SARS-CoV-2), insbesondere wobei der Wirkstoff in die Nasenhöhle des Subjekts verabreicht wird, insbesondere wobei der Wirkstoff für die topische Verabreichung formuliert ist, insbesondere wobei die topische Formulierung eine Nasencreme, Nasentropfen oder ein Nasenspray umfasst.

14. Zusammensetzung zur Verwendung gemäß Anspruch 13, wobei die Verabreichung die Fähigkeit von SARS-CoV-2 verringert, in das Nasenepithel einzudringen, sich im Nasenepithel zu vermehren und/oder aus dem Nasenepithel zu wandern.

15. Eine Gesichtsmaske, umfassend eine Bahn aus einem Filtermaterial und einen Wirkstoff, ausgewählt aus Cysteamin, Cystamin und Phosphocysteamin, der in die Bahn eingearbeitet ist.

## Revendications

1. Composition comprenant une dose efficace d'un agent actif unique choisi parmi la cystéamine, la cystamine et la phosphocystéamine, en tant qu'antiviral destiné à être utilisé dans le traitement du coronavirus 2 du syndrome respiratoire aigu sévère (SARS-CoV-2) chez un sujet.

2. Composition destinée à être utilisée selon la revendication 1, dans laquelle le sujet est atteint de la maladie à coronavirus 2019 (COVID-19), en particulier dans laquelle le sujet souffre d'une forme légère de COVID-19, ou en particulier dans laquelle le sujet souffre d'une forme modérée de COVID-19, ou en particulier dans laquelle le sujet souffre d'une forme grave de COVID-19 ; dans laquelle la forme légère de COVID-19 est **caractérisée par** un ou plusieurs des symptômes suivants : fièvre faible ou modérée, symptômes respiratoires, toux, courbatures et douleurs ; dans laquelle la forme modérée de COVID-19 est **caractérisée par** la présence d'infiltrats pulmonaires sans nécessiter d'oxygénothérapie, toux, une fièvre supérieure à 37,8 °C, des frissons, un essoufflement, une déshydratation et une fatigue accrue ; la forme grave de la COVID-19 étant **caractérisée par** un ou plusieurs des symptômes suivants : inflammation pulmonaire, pneumonie, faible taux d'oxygène dans le sang, difficultés respiratoires, douleur ou pression thoracique, confusion, incapacité à se réveiller, difficulté à manger ou à boire, et coloration bleuâtre des lèvres ou du visage.

3. Composition destinée à être utilisée selon l'une des revendications 1 à 2, dans laquelle la dose efficace comprend 10 à 500 mg d'agent actif, en particulier dans laquelle la dose efficace comprend 25 à 150 mg d'agent actif.

4. Composition destinée à être utilisée selon l'une des revaendications 1 à 3, dans laquelle l'agent actif est administré quotidiennement, en particulier dans laquelle l'agent actif est administré deux fois par jour.

5. Composition destinée à être utilisée selon l'une des revendications 1 à 4, dans laquelle l'agent actif est administré par voie orale, et/ou dans laquelle l'agent actif est administré par voie intraveineuse.

6. Composition destinée à être utilisée selon l'une des revendications 1 à 5, dans laquelle la dose efficace comprend 25 à 250 mg/kg/jour d'agent actif, et/ou dans laquelle la dose quotidienne ne dépasse pas 20 g/jour d'agent actif, et/ou dans laquelle le sujet reçoit d'abord une dose initiale, puis les doses sont augmentées jusqu'à atteindre une dose efficace.

7. Composition destinée à être utilisée selon l'une des revendications 1 à 6, dans laquelle le sujet a fait l'objet d'un test visant à déterminer la pertinence de l'administration de cystéamine pour traiter ou prévenir la COVID-19, et dans laquelle, si cela s'avère approprié, une dose d'un agent actif est administrée au sujet.

8. Composition destinée à être utilisée selon la revendication 7, dans laquelle le test comprend un test par PCR visant à déterminer si le sujet est infecté par le SARS-CoV-2.

9. Composition destinée à être utilisée selon la revendication 7, dans laquelle le sujet est soumis à un test visant à déterminer l'efficacité de l'administration de l'agent actif.

10. Substance comprenant un agent actif unique choisi parmi la cystéamine, la cystamine et la phosphocystéamine en tant qu'antiviral, destinée à être utilisée dans un procédé de traitement d'un sujet infecté par le coronavirus 2 du syndrome respiratoire aigu sévère (SARS-CoV-2), la forme grave de la COVID-19 étant **caractérisée par** un ou plusieurs des symptômes suivants : inflammation pulmonaire, pneumonie, faible taux d'oxygène dans le sang, difficultés respiratoires, douleur ou pression thoracique, confusion, incapacité à se réveiller, difficulté à manger ou à boire, et coloration bleuâtre des lèvres ou du visage.

11. Substance destinée à être utilisée selon la revendication 10, dans laquelle la mise en contact de la protéine Spike du SARS-CoV-2 avec l'agent actif modifie la structure de ladite protéine Spike, en particulier dans laquelle la mise en contact de la protéine Spike avec l'agent actif entraîne la réduction d'un ou plusieurs ponts disulfure au sein de la protéine Spike.

12. Substance destinée à être utilisée selon la revendication 11, dans laquelle la protéine Spike est présente à la surface d'une particule virale du SARS-CoV-2, en particulier dans laquelle la particule virale du SARS-CoV-2 est présente chez un sujet humain.

13. Composition destinée à être utilisée comme médicament, comprenant une dose efficace d'un agent actif unique choisi parmi la cystéamine, la cystamine et la phosphocystéamine, en tant qu'antiviral destiné à être utilisé dans la prévention de l'infection d'un sujet par le coronavirus 2 du syndrome respiratoire aigu sévère (SARS-CoV-2), en particulier dans laquelle l'agent actif est administré dans la cavité nasale du sujet, en particulier dans laquelle l'agent actif est formulé pour une administration topique, en particulier dans laquelle la formulation topique comprend une crème nasale, des gouttes nasales ou un spray nasal.

14. Composition destinée à être utilisée selon la revendication 13, dans laquelle ladite administration réduit la capacité du SARS-CoV-2 à pénétrer dans l'épithélium nasal, à se répliquer dans l'épithélium nasal et/ou à migrer à partir de l'épithélium nasal.

15. Masque facial comprenant un panneau en matériau filtrant et un agent actif choisi parmi la cystéamine, la cystamine et la phosphocystéamine, incorporé dans le panneau.
